Europäisches Patentamt

European Patent Office

Office européen des brevets

(19)

(11) **EP 0 409 176 B1**

(12) **EUROPEAN PATENT SPECIFICATION**

(45) Date of publication and mention
of the grant of the patent:
**28.04.1999 Bulletin 1999/17**

(51) Int Cl.$^6$: **G01N 33/74**

(21) Application number: **90113694.5**

(22) Date of filing: **17.07.1990**

(54) **Method and materials for detecting pathology from alterations in estrogen metabolism**

Verfahren und Mittel zum Nachweis von Krankheitszuständen aus Änderungen im Östrogenstoffwechsel

Procédés et réactifs pour la détection des maladies à cause des altérations en métabolisme estrogène

(84) Designated Contracting States:
**AT BE CH DE DK ES FR GB GR IT LI LU NL SE**

(30) Priority: **17.07.1989 US 381064**
**11.07.1990 US 549290**

(43) Date of publication of application:
**23.01.1991 Bulletin 1991/04**

(73) Proprietor: **THE ROCKEFELLER UNIVERSITY**
**New York New York 10021-6399 (US)**

(72) Inventors:
• **Michnovicz, Jon J.**
**Brooklyn, NY 11230 (US)**
• **Hershcopf, Richard J.**
**Tenafly, NJ 07670 (US)**
• **Bradlow, H. Leon**
**Holliswood, NY 11423 (US)**
• **Fishman, Jack**
**Miami, FL 33156 (US)**

(74) Representative: **Jorio, Paolo et al**
**STUDIO TORTA S.r.l.,**
**Via Viotti, 9**
**10121 Torino (IT)**

(56) References cited:
**EP-A- 0 046 004        EP-A- 0 179 037**
**EP-A- 0 200 960**

• STEROIDS, vol. 52, nos. 1-2, August 1988, Stoneham, MA (US); J.J. MICHNOVICZ et al., pp. 69-83#
• METABOLISM, vol. 38, no. 6, June 1989; J.J. MICHNOVICZ et al., pp. 537-541#

• NEW ENGLAND JOURNAL OF MEDICINE, vol. 315, no. 21, 20 November 1986, Boston, MA (US); J.J. Michnovicz et al., pp. 1305-1309#
• JOURNAL OF STEROID BIOCHEMISTRY, vol. 24, no. 1, 1986, Oxford (GB); H. ADLERCREUTZ et al., pp. 289-296#
• AMERICAN JOURNAL OF EPIDEMIOLOGY, vol. 119, no. 1, January 1984, Baltimore, MD (US); J.A. BARON, pp. 9-22#
• STEROIDS, vol. 55, no. 1, January 1990, Stoneham, MA (US); J.J. MICHNOVICZ et al., pp. 22-26#
• NEW ENGLAND JOURNAL OF MEDICINE, vol. 321, no. 5, 03 August 1989, Boston, MA (US); R.A. GALBRAITH et al., pp. 269-274#
• EUR. J. CANCER CLIN. ONC., vol.24, no.7, 1988, page 1171 - 1178, Y.J. ABUL-HAJJ ET AL. 'METABOLISM OF ESTRADIOL BY HUMAN BREAST CANCER'
• J. STEROID BIOCHEM., vol.34, no.1-6, 1989, page 527 - 530, H. ADLERCREUTZ ET AL. 'DIET AND URINARY ESTROGEN PROFILE IN PREMENOPAUSAL OMNIVOROUS AND VEGETARIAN WOMEN AND IN PREMENOPAUSAL WOMEN WITH BREAST CANCER'
• ANNALS N Y. ACAD. SCI., vol., no., 1990, page 252 - 258, J.H.H. THIJSSEN AND M.A. BLANKENSTEIN 'UPTAKE AND METABOLISM OF ESTRADIOL BY NORMAL AND ABNORMAL BREAST TISSUES'
• J. NATL. CANC. INST. , vol.86, no.14, 20.07.94, page 1076 - 1082, H. ADLERCREUTZ ET AL. 'ESTROGEN METABOLISM AND EXCRETION IN ORIENTAL AND CAUCASIAN WOMEN'

EP 0 409 176 B1

Remarks:

The file contains technical information submitted after the application was filed and not included in this specification

**Description**

The Applicants are authors or co-authors of the following articles directed to the subject matter of the present invention: (1) [Applicants Michnovicz, Hershcopf, Bradlow and Fishman are co-authors with Hiroshi Naganuma] "Increased Urinary Catechol Estrogen Excretion In Female Smokers", STEROIDS 52/1-2 at 69-83 (July-August, 1988); (2) [Applicants co-authored with Nancy J. Haley] "Cigarette Smoking Alters Hepatic Estrogen Metabolism in Men: Implications for Atherosclerosis", METABOLISM 38 (6) at 537-541 (June, 1989); (3) [Applicant Michnovicz co-authored with Richard A. Galbraith] "The Effects of Cimetidine on the Oxidative Metabolism of Estradiol", N. ENGL. J. MED., 321/5:269-274 (August 3, 1989); (4) [Applicant Michnovicz co-authored with Richard A. Galbraith] "Effects of Exogenous Thyroxine on C-2 and C-16 Hydroxylations of Estradiol in Humans", STEROIDS, 55:22-26 (January, 1990); and (5) [Applicants Michnovicz and Bradlow co-authored] "Induction of Estradiol Metabolism by Dietary Indole-3-Carbinol in Humans", J. NATL. CANCER INS. (Published June, 1990).

The present invention relates to the diagnosis of pathology in mammals, and particularly to the comparison of the levels of estrogen metabolites and the correlation of metabolite levels with the onset or presence of various pathological conditions in mammals and particularly in humans.

The metabolism of estrogen has been the subject of numerous studies seeking to correlate the presence of particular pathologies such as endometrial cancer and breast disease, with the presence and amount of certain estrogen metabolites. Thus, studies have focused on metabolites of estrone such as $2\alpha$-hydroxyestrone, estradiol and estriol, and have speculated to some extent as to the role, if any, that these metabolites might play in certain pathological scenarios. Particularly in the instance of $16\alpha$-hydroxyestrone, these studies have suggested a direct pathophysiological involvement. See, for example, Fishman et al., J. CLIN. ENDOCRINOL. METAB. 51(3):611-615 (1980). However, the constitutive nature of this metabolite has discouraged its further consideration for either diagnostic or therapeutic purposes.

At present, the diagnosis of the above pathological conditions and abnormalities is generally performed after the patient has experienced some abnormal physical response, e.g., lack of energy, headaches, rectal bleeding, lumps, etc., or as preliminarily detected during an annual physical examination. Once evidencing such abnormal physical response, diagnostic procedures and/or other protocols are thereafter initiated and evaluated to qualify the pathological state as well as to quantify the extent of advancement of the pathological state or condition. Diagnostic procedures may involve X-rays, e.g., mammography for breast cancer, etc.

Additionally, once a pathological state has been found to exist and has been qualified as to the specific pathological state, there may be remedial procedures to reduce the impact of the pathological state, such as drug, radiation therapy, chemotherapy, dietary and other lifestyle factors, and the like protocol, or alternately, to eliminate the pathological state, e.g., by surgical procedure. In any event, the effectiveness of the remedial procedure is difficult to timely assess. For example, in the surgical removal of cancerous growth, only subsequent biopsies of proximate tissue may demonstrate total removal, and then, not necessarily on a 100 percent certain basis, let alone the possibility of metastasis.

Tests have been developed wherein a cellular sample is isolated and tested for individual functionality by diverse methods. Such procedures are costly and time-consuming and are not specific to a particular pathological state. Also, the results of such tests are difficult to interpret, let alone correlate. For example, although mammography may delineate the size, location, etc., of a lump in the breast in a female, the results will not always qualify whether the lump is cancerous or benign. Such pathological evaluation is effected by pathological observation of the actual cellular structure after biopsy or surgical removal of the lump.

While disease-associated changes in estrogen metabolism have been noted earlier, the nature of the metabolites has discouraged efforts to apply this phenomenon toward the development of a simple diagnostic test. This is because the level of estrogen and its metabolism fluctuates in response to the menstrual cycle in females as well as to circadian rhythm in both men and women. The need continues to exist for the development of a safe and preferably non-invasive, reliable and inexpensive test for the assessment of the above and other pathological states and abnormalities wherein the metabolism of estrogen may be implicated.

*The article by Michnovicz et al., Steroids, volume 52, pp. 69-83, (August 1988), "Increased Urinary Catechol Estrogen Excretion in Female Smokers" was co-authored by several of the instant inventors, and concerns a study using radiometric assays wherein the steroid to be metabolized was exogenously produced, radiolabelled, administered to the test subjects so as to undergo metabolism, and then analyzed.*

*Michnovicz et al., Metabolism, Volume 38, No. 6, June 1989, "Cigarette Smoking alters hepatic estrogen metabolism in men: Implications for atherosclerosis" is a further publication co-authored by certain of the inventors of the instant Application. As is the case with the above-mentioned Steroid reference, this study involved the use of radiolabelled, exogenously administered estradiol and its metabolism by smoking men.*

*Michnovicz et al., New England Journal of Medicine, Volume 315, No. 21, (1986) "Increased 2-hydroxylation of estradiol as a possible mechanism for the antiestrogenic effect of cigarette smoking", is again a publication co-*

*authored by the certain of the inventors of the instant invention, and again, deals with the analysis of exogenously administered radiolabelled estradiol and its metabolites.*

*Adlercreutz et al., Journal of Steroid Biochemistry, Volume 24, No. 1, 1986, "Urinary estrogen profile determination in young Finnish vegetarian and omnivorous women", deals with studies conducted on healthy individuals with varying dietary intake However, the determinations of Adlercreutz were all made with healthy individuals so that the results could not be utilized as a diagnosis of a pathology since none was observed during the studies. Secondly, the results of Adlercreutz would seem to indicate that estrogen profile determination could not be used to diagnose a pathology since the study determined that dietary fiber intake significantly affects estrogen metabolism. Although Adlercreutz mentions in the abstract that "..diet may influence estrogen metabolism and in this way affect breast cancer risk..", his results in no way support the use of a ratio of estrone metabolites to diagnose pathologies such as breast cancer.*

*On the contrary, a method and associated materials for detecting pathology from alterations in estrogen metabolism in mammals are hereby disclosed.* The method comprises isolating at least two distinct metabolites of estrone from a biological sample taken from the mammal under examination, determining the quantity of each of the said metabolites in the sample, correlating the quantities of each of said metabolites with each other to arrive at a quotient of said metabolites, and comparing said quotient with an extrinsic quotient derived either previously from the mammal under test, as by the previous performance of the within test, or from the testing of other subjects of the same species, to detect any alterations in said estrogen metabolism.

*This determining or the quantity of each of the said metabolites in the sample may be by measurement of the concentration of said products in the sample, and this concentration measurement may be accomplished by chromatographic, immunochemical, or radiometric analysis or by a receptor assay.* The metabolites of estrone include products of enzymatic hydroxylation, products of enzymatic reduction of estrone, products of enzymatic oxidation of estrone and products of enzymatic methylation of estrone. The metabolites of estrone broadly comprise 2-hydroxyestrone, 4-hydroxyestrone, 15α-hydroxyestrone, 16α-hydroxyestrone, 2-methoxyestrone, and estriol, 17β-estriol, epiestriol, 16-ketoestradiol, and 15α-hydroxyestradiol, and more particularly, consist essentially of 2-hydroxyestrone and estriol.

As used herein, the term "hydroxylation products of estrone" applies not only to the various hydroxyl-substituted estrones, but to subsequent metabolites thereof, as may be formed, for example, by the oxidation or reduction of the substituted estrones with the enzyme 17-oxidoreductase.

In a particular embodiment, the present method comprises the quantitation of the levels of the products of enzymatic estrone hydroxylation, and particularly the levels of 2-hydroxyestrone ($2OHE_1$) and estriol ($E_3$), and the determination of a quotient or index defined by the formula $[2OHE_1]/[E_3]$.

The use of the Index determined by the method of the present invention eliminates the need for corrections to accommodate biological variations as between subjects, as well as variations due to the menstrual cycle in female subjects, and circadian rhythm in both male and female subjects. The present method thereby provides the basis for diagnostic tests offering clinically significant data with reduced procedural complexity.

The present method and the specific protocols that may be derived therefrom as set forth below and later on herein, are useful for detecting pathologies in which an alteration in estrogen metabolism is implicated, such as autoimmune diseases, liver diseases, osteoporosis, endometriosis, obesity, atherosclerosis, endometrial cancer, breast cancer and heart disease. Likewise, the present method and associated test kits may be used to monitor the course of therapy, or test the efficiency of new drugs and the like.

Accordingly, the present invention also relates to a method for determining the presence of stimulated, spontaneous, or idiopathic pathological states in mammals, by measuring the activity and presence of the noted metabolites of estrone. More particularly, the quantity or activity of the metabolites of estrone may be followed directly by the assay techniques discussed later on, through the use of an appropriately labeled quantity of the metabolites. Alternately, the estrone metabolites can be used to raise binding partners or antibodies that could in turn, be labeled and introduced into a medium such as serum, to test for the presence of metabolites of estrone therein, and to thereby assess the state of the host from which the medium was drawn.

Thus, both the metabolites of estrone and any antibodies that may be raised thereto, are capable of use in connection with various diagnostic techniques, including immunoassays, such as a radioimmunoassay, using for example, an antibody to the hydroxylation products that has been labeled by either radioactive addition, reduction with sodium borohydride, or radioiodination.

In an exemplary immunoassay, a control quantity of an estrone metabolite, its antibody, or the like may be prepared and labeled with an enzyme, a specific binding partner and/or a radioactive element, and may then be introduced into a urine, blood, saliva or other physiological fluid sample of a mammal believed to be in a pathological state. After the labeled material or its binding partner(s) has had an opportunity to react with sites within the sample, the resulting mass may be examined by known techniques, which may vary with the nature of the label attached.

In the instance where a radioactive label, such as the isotopes $^{14}C$, $^{131}I$, $^{3}H$, $^{125}I$, $^{57}Co$, $^{59}Fe$ and $^{35}S$ are used, known currently available counting procedures may be utilized. In the instance where the label is an enzyme, detection may be accomplished by any of the presently utilized colorimetric, spectrophotometric, thermometric, amperometric, fluorospectro-photometric or gasometric techniques known in the art.

The materials developed and useful in accordance with the present invention includes an assay system which may be prepared in the form of a test kit for the quantitative analysis of the extent of the presence of the estrone metabolite. The system or test kit may comprise a labeled component prepared by one of the radioactive and/or enzymatic techniques discussed herein, directly or indirectly coupling a label to the estrone metabolite; and one or more additional immunochemical reagents, at least one of which is a free or immobilized ligand, capable either of binding with the labeled component, its binding partner, one of the components to be determined or their binding partner; said estrone metabolites selected so as to compare the 2-hydroxylation and 16α-hydroxylation pathways of estrone metabolism.

Accordingly, it is a first object of the present invention to provide a method for determining whether a pathological state or condition exists in a mammal.

It is a further object of the present invention to provide a method as aforesaid exists that may be performed in a facile and inexpensive manner.

It is a yet further object of the present invention to provide a dependable method for determining whether a pathological state or condition exists in a mammal with minimal, if any, false readings.

A still further object of the present invention is to provide a simple method for sequentially determining the course of a known pathological state or condition in a mammal.

Still another object of the present invention is to provide a method for determining the effectiveness of a surgical procedure on a mammal to eradicate a pathological state or condition, or to detect recurrent disease.

A still further object of the present invention is to provide a method for monitoring the effectiveness of a drug or dietary regime or like protocol on a mammal having a known pathological state or condition.

A still further object of the present invention is to provide a method for determining the reproductive status or the course of pregnancy in a mammal. Other objects and advantages will become apparent to those skilled in the art from a review of the ensuing description which proceeds with reference to the following illustrative drawings.

FIGURE 1A is a flow diagram depicting the metabolic pathways of estrone.

FIGURE 1B is a flow diagram depicting the metabolic pathways of estradiol.

FIGURES 2A and 2B are graphs of the results of the measurement of 2-hydroxylation of estrone as between female smokers and nonsmokers. FIGURE 2A is a scattergram presentation of the ratios determined in accordance with the present invention, and FIGURE 2B represents confirmatory radiometric measurements of the same groups compared with the ratio data presented in FIGURE 2A.

FIGURES 3A and 3B are graphs of the results of radiometric measurements of the percentage of estradiol 2-hydroxylation of groups of male smokers and nonsmokers. FIGURE 3A depicts the radiometric measurements and FIGURE 3B depicts the comparison of ratios from the same groups.

FIGURE 4 is a graph plotting the radiometric extent of 2-hydroxylation versus urinary $[2OHE_1]/[E_3]$ for each individual, showing a strong correlation between the two measures ($r = 0.71, P < .002$). The line represents the least squares fit to the points.

The present invention relates to a method and associated materials for detecting pathology from alterations in estrogen metabolism in mammals. The present method comprises isolating at least two distinct metabolites of estrone from a biological sample taken from the mammal under examination, said estrone metabolites selected so as to compare the 2-hydroxylation and 16α-hydroxylation pathways of estrone metabolism, determining the quantity of each of said metabolites in said sample, correlating the ratio of the quantities of each of said metabolites with each other to arrive at a ratio of said metabolites and comparing said ratio with an extrinsic ratio derived either previously from the mammal under testing, or from the testing of other subjects of the same species, to detect any alterations in said estrogen metabolism, said alterations identifying to existence of said pathology.

The metabolites of estrone that may be measured and monitored in accordance with the present invention include and may be selected from the products of enzymatic hydroxylation of estrone, the products of the enzymatic reduction of estrone, the products of the enzymatic oxidation of estrone and the products of the enzymatic methylation of estrone. As mentioned earlier, products of enzymatic hydroxylation of estrone include such hydroxylated estrones as may be further enzymatically oxidized or reduced, as by the action of 17-oxidoreductase.

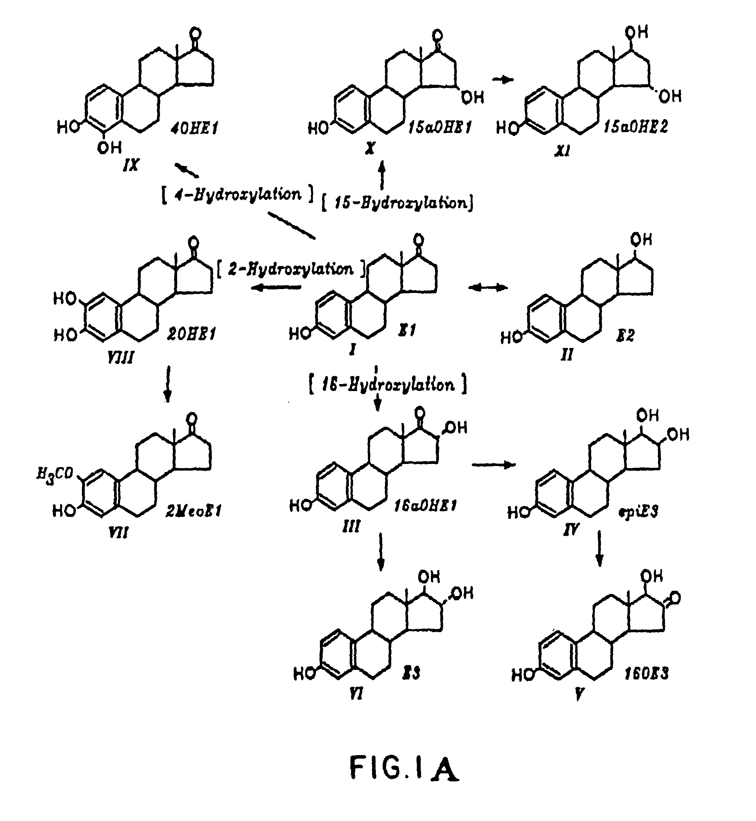

Accordingly, and as depicted in FIGURE 1A, the estrone metabolites of interest herein include 2-hydroxyestrone, 4-hydroxyestrone, 15α-hydroxyestrone, 16α-hydroxyestrone, 2-methoxyestrone, estriol, 17β-estriol, epiestriol, 16-ketoestradiol, and 15α-hydroxyestradiol. The products of enzymatic hydroxylation broadly comprise 2-hydroxyestrone, 16α-hydroxyestrone, 2-methoxyestrone, and estriol, and more particularly, 2-hydroxyestrone and estriol.

More particularly, the present invention comprises the measurement and quantitation of the products of enzymatic estrone hydroxylation, and the determination of an algorithmic relationship between certain of these hydroxylation products that provides a reliable indication of pathological condition.

Accordingly, in one embodiment a ratio or quotient may be determined between the metabolites 2-hydrox-

yestrone ($2OHE_1$) and estriol ($E_3$), that is expressed as

$$[2OHE_1]/[E_3]$$

and which may be compared with either prior or subsequent such ratios taken from the measurement of the respective levels of these estrogen metabolites in the same subject, or in subjects of the same species. In the former instance, the physician or the patient may periodically monitor the index to note any changes that would suggest the desirability of more specific testing for one of the conditions or pathologies that are known to cause fluctuations in estrogen metabolism. In the latter instance, the individual index or quotient may be compared with a continuum of similar data developed from subjects both healthy and afflicted with specific pathologies to attempt to identify in an approximate qualitative fashion, the pathological state of the particular subject under test.

As stated earlier herein, the theory of the invention was derived in part from studies of estrogen metabolism in smokers. Several epidemiological studies have indicated that cigarette smoking substantially alters the turnover of estrogen in women (Baron, A. J., AM. J. EPIDEMIOL., 119:9-22 (1984)). Female smokers have been found to have decreased endometrial cancer (Weiss, N. S., et al., MATURITAS, 2:185-190 (1980); Tyler, C. W., et al., AM. J. OBSTET. GYNECOL., 151:899-905 (1985); Lesko, S. M., et al., N. ENGL. J. MED., 313:593-596 (1985); Baron, J. A., et al., JNCI, 77:677-680 (1986)), decreased endometriosis (Cramer, D. W., et al., JAMA, 255:1904-1908 (1986)), decreased benign breast disease (Berkowitz, G. S., et al., J. EPIDEMIOL. COMMUNITY HEALTH, 39:308-313 (1985)), increased osteoporosis (Daniell, H. W., ARCH. INTERN. MED., 136:298-304 (1976); Jensen, J., et al., N. ENGL. J. MED., 313: 973-975 (1985)), and earlier natural menopause (Willett, W. L., AM. J. EPIDEMIOL., 117:651-658 (1983)).

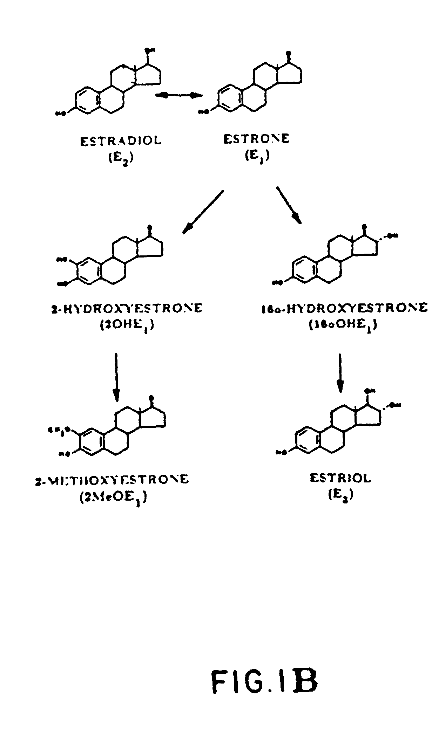

It was recently reported that smoking is associated with a significant increase in estradiol 2-hydroxylation as measured by an in vivo radiometric procedure in premenopausal females (Michnovicz, J. J., et al., N. ENGL. J. MED., 315:1305-1309 (1986)). This major irreversible biotransformation is depicted in the estrogen metabolic reaction pathway of FIGURE 1B and follows the conversion of estradiol ($E_2$) to estrone ($E_1$ and yields the catechol estrogens 2-hydroxyestrone ($2OHE_1$) and 2-methoxyestrone ($2MeOE_1$), both possessing minimal peripheral estrogenic activity (Fishman, J., et al., J. BIOL. CHEM., 235:3104-3107 (1960); Martucci, C. P., et al, ENDOCRINOLOGY, 105:1288-1292 (1979); Jellinck, P. H., et al., ENDOCRINOLOGY, 108:1848-1854 (1981)).

The present invention also extends to the quantitation and analysis of other pathways including the 4-hydroxylation, 15-hydroxylation and 16α-hydroxylation pathways, which 16α-hydroxylation results in the formation of 16α-hydroxyestrone ($16\alpha OHE_1$) and estriol ($E_3$), which retain estrogen agonist activity (Fishman, J., et al., J. CLIN. ENDOCRINOL. METAB., 51:611-615 (1980)). The specific enzymes involved in the reaction pathway of FIGURE 1A include 17-oxidoreductase which is implicated in the conversion of estradiol to estrone, 16α-hydroxyestrone to estriol, 15α-hydroxyestrone to 15α-hydroxyestradiol, and of epiestriol to 16-ketoestriol; 2-hydroxylase implicated in the hydroxylation of estrone to 2-hydroxyestrone; catechol-O-methyltransferase, involved in the conversion of 2-hydroxyestrone to 2-methoxyestrone; 15α-hydroxylase which is involved in the hydroxylation of estrone to 15α-hydroxyestrone; 4-hydroxylase which is involved in the production of 4-hydroxyestrone; and 16α-hydroxylase, which is involved in the hydroxylation of estrone to 16α-hydroxyestrone.

A similar relationship was postulated to exist in the male mammalian population, as, for example, men have a higher mortality rate from ischemic heart disease than age-matched women (Kuller, L. H., et al., AM. J. EPIDEMIOL., 104:425-426 (1976); Heller, R. F., et al., BR. MED. J., 1:472-474 (1978); Kannel, W. B., et al., ANN. INTERN. MED., 85:477-452 (1976); Bush, T. L., et al., EPIDEMIOL. REV., 7:80-104 (1985)). In addition, the incidence of heart disease increases significantly in women after menopause, coincident with the decline in ovarian estrogen production (Kannel, W. B., et al., ANN. INTERN. MED. 85:477-452 (1976)). These epidemiologic data point to estrogen as a protective factor against heart disease (Bush, T. L., et al., EPIDEMIOL. REV., 7:80-104 (1985)). Premenopausal females have greater mean high-density lipoprotein-cholesterol (HDL-C) than age-matched males (Rifkind, B. M., et al., LIPIDS, 14: 105-112 (1979); Conner, S. L., et al., CIRCULATION, 65:1290-1298 (1980); Wahl, P. W., et al., ATHEROSCLEROSIS, 39:111-124 (1981)), except under such androgenizing conditions as polycystic ovary syndrome (Wild, R. A., et al., J. CLIN. ENDOCRINOL. METAB., 61:946-951 (1985). Administration of estrogens to both men and women produces favorable changes in lipoproteins, with increased HDL-C and decreased low-density lipoprotein-cholesterol (LDL-C) and total cholesterol.

As a result of the above and as confirmed by the results of the experiments set forth in Examples I-V, below, it has been determined that the index or quotient of the products of enzymatic estrogen metabolism varies with the presence and extent of pathology and is sufficiently independent of other systemic factors that it can serve as a reliable indicator of pathological status.

The pathological conditions, the nonspecific existence of which are identified by the present invention include osteoporosis, endometriosis, obesity, atherosclerosis, endometrial cancer, breast cancer, heart disease, myocardial infarction, trauma, vascular thrombosis, infertility, gastrointestinal disease, autoimmune disease, central nervous system disease, etc., and any pathological state or condition affecting the estrogen metabolism of a mammal, it being understood by one skilled in the art that the specific pathological state or condition existing in a test mammal is generally qualified after a positive determination of the existence of a pathological state or condition in accordance with the method of the present invention. The term "mammals" as used herein includes homo sapiens as well as domesticated animals, e.g., race horses.

The present invention also relates to a variety of diagnostic applications, including methods for determining the presence of disease by reference to the ability to detect alterations in estrogen metabolism which are affected by the presence of various pathological conditions in mammals. Also, the present method may be employed to monitor those events in which the level of estrogen metabolites is directly implicated, such as the fertility of the male and female of the species and the course of pregnancy. The present method may therefore be employed as an adjunct to therapy for impotence or infertility, or with respect to possible irregularities during pregnancy, the female menstrual cycle, or at the onset or during menopause. As mentioned earlier, the metabolites of estrone can be used to produce antibodies by a variety of known techniques, and such antibodies could then be isolated and utilized as in tests for alterations in estrogen metabolism in suspect mammals.

Antibody(ies) to the metabolites of estrone can be produced and isolated by standard methods including the well known hybridoma techniques. Both polyclonal and monoclonal antibodies are included; however, monoclonal antibodies are preferred. Monoclonal antibodies may be prepared in accordance with the teachings of Kohler, G., et al., NATURE 256:495-497 (1975), which reference is cited herein by way of illustration only, and not by way of limitation. In particular, antibodies may be prepared that cross-react with more than one metabolite, and, for example, a monoclonal antibody could be prepared that would bind to $E_3$ and $16OHE_1$ for use in determining ratios that are based in part on $16OHE_1$ and $E_3$. For convenience, the antibody(ies) to the metabolites of estrone will be referred to herein as $Ab_1$ and antibody(ies) raised in another species as $Ab_2$.

The presence of metabolites of estrone in mammals can be ascertained by the usual immunological procedures applicable to such determinations. A number of useful procedures are known. Four such procedures which are especially useful utilize either the metabolite of estrone labeled with a detectable label, antibody $Ab_1$ labeled with a detectable label, or antibody $Ab_2$ labeled with a detectable label or a chemical conjugate with the hydroxylation product of estrone labeled with a detectable label. The procedures may be summarized by the following equations wherein the asterisk indicates that the particle is labeled, and "OHE" in this instance stands for all metabolites of estrone:

A $OHE* +'Ab: = OHE*Ab_1$
B $OHE + Ab* = OHEAb_1*$
C $OHE + Ab_1 + Ab_2* = OHEAb_1Ab_2*$
D $Carrier*OHE + Ab_1 = Carrier*OHEAb_1$

The procedures and their application are all familiar to those skilled in the art and are presented herein as illustrative and not restrictive of procedures that may be utilized within the scope of the present invention. The "competitive" procedure, Procedure A, is described in U.S. Patent Nos. 3,654,090 and 3,850,752. Procedure C, the "sandwich" procedure, is described in U.S. Patent Nos. RE 31,006 and 4,016,043. Still other procedures are known such as the "double antibody", or "DASP" procedure. A further diagnostic procedure employs multiple labeled compounds in a single solution for simultaneous radioimmune assay. In this procedure disclosed in U.S. Patent No. 4,762,028 to Olson, a composition may be prepared with two or more analytes in a coordinated compound having the formula: radioisotope-chelator-analyte.

In each instance, the hydroxylation product of estrone forms complexes with one or more antibody(ies) or binding partners and one member of the complex is labeled with a detectable label. The fact that a complex has formed and, if desired, the amount thereof, can be determined by known methods applicable to the detection of labels.

It will be seen from the above, that a characteristic property of $Ab_2$ is that it will react with $Ab_1$. This is because antibodies raised in one mammalian species have been used in another species as an antigen to raise antibodies such as $Ab_2$. For example, $Ab_2$ may be raised in goats using rabbit antibodies as antigens. $Ab_2$ therefore would be anti-rabbit antibody raised in goats. For purposes of this description and claims, $Ab_1$ will be referred to as a primary or anti-estrogen metabolite antibody, and $Ab_2$ will be referred to as a secondary or anti-$Ab_1$ antibody.

The labels most commonly employed for these studies are radioactive elements, enzymes, chemicals which fluoresce when exposed to ultraviolet light, and others.

A number of fluorescent materials are known and can be utilized as labels. These include, for example, fluorescein, rhodamine and auramine. A particular detecting material is anti-rabbit antibody prepared in goats and conjugated with fluorescein through an isothiocyanate.

The hydroxylated products of estrone or carrier of same, or their binding partner(s) can also be labeled with a radioactive element or with an enzyme. The radioactive label can be detected by any of the currently available counting procedures. The preferred isotope may be selected from $^{14}C$, $^{131}I$, $^{3}H$, $^{125}I$, $^{57}Co$, $^{59}Fe$ and $^{35}S$.

Enzyme labels are likewise useful, and can be detected by any of the presently utilized colorimetric, spectrophotometric, fluorospectrophotometric, thermometric, amperometric or gasometric techniques. The enzyme is conjugated to the estrogens or their binding partners or carrier molecules by reaction with bridging molecules such as carbodiimides, diisocyanates, glutaraldehyde and the like.

Many enzymes which can be used in these procedures are known and can be utilized. The preferred are peroxidase, β-glucuronidase, β-D-glucosidase, β-D-galactosidase, urease, glucose oxidase plus peroxidase, *galactose oxidase plus peroxidase,* hexokinase plus GPDase, glucose oxidase plus alkaline phosphatase, NAD oxidoreductase plus luciferase, phosphofructokinase plus phosphoenol pyruvate carboxylase, aspartate aminotransferase plus phosphoenol pyruvate decarboxylase, and alkaline phosphatase. U.S. Patent Nos. 3,654,090; 3,850,752; and 4,016,043 are referred to by way of example for their disclosure of alternative labeling material and methods.

A particular assay system developed and utilized in accordance with the present invention, is known as a receptor assay. In a receptor assay, the material to be assayed is appropriately labeled and then certain cellular test colonies are inoculated with a quantity of both the labeled and unlabeled material after which binding studies are conducted to determine the extent to which the labeled material binds to the cell receptors. In this way, differences in affinity between materials can be ascertained.

Accordingly, a purified quantity of the hydroxylated products of estrone may be radiolabeled, after which binding studies would be carried out using for example, MCF-7 breast cancer cells (ATCC Accession No. HTB 22). Solutions would then be prepared that contain various quantities of labeled and unlabeled MCF-7 cell samples would then be inoculated and thereafter incubated. The resulting cell monolayers would then be washed, solubilized and then counted in a scintillation counter for a length of time sufficient to yield a standard error of <5%. These data are then subjected to Scatchard analysis after which observations and conclusions regarding material activity can be drawn. While the foregoing protocol is exemplary, it illustrates the manner in which a receptor assay may be performed and utilized, in the instance where the cellular binding ability of the assayed material may serve as a distinguishing characteristic.

A variation of the exemplary receptor assay presented above is disclosed in U.S. Patent No. 4,818,684. In this assay, a competitive protocol is used which employs monoclonal antibodies capable of binding to a steroid receptor which in turn are competitively bound by anti-steroid antibodies capable of binding to the steroid. The assay proceeds by the competition between the anti-steroid antibodies and the receptor. The presence or amount of monoclonal antibody anti-steroid antibody complex thus formed is related to the amount of steroid receptor present in the assayed material.

In a further embodiment of this invention, commercial test kits suitable for use by a medical specialist may be prepared to determine the presence or absence of hydroxylation products of estrone in a suspected mammal. For example, one class of such kits will contain at least a labeled component selected from the hydroxylation products of estrone or their binding partners, for instance an antibody specific thereto, and directions, of course, depending upon the method selected, e.g., "competitive", "sandwich", "DASP" and the like. The kits may also contain peripheral reagents such as buffers, stabilizers, etc. Accordingly, a test kit may be prepared for the demonstration of the altered estrogen metabolism of a mammalian host, comprising:

(a) a predetermined amount of at least one labeled immunochemically reactive component obtained by the direct or indirect attachment of the present metabolites of estrone or a specific binding partner thereto, to a detectable label, said estrone metabolites selected so as to compare the 2-hydroxylation and 16α-hydroxylation pathways of estrone metabolism;
(b)- other reagents; and
(c)- direction for use of said kit.

More specifically, the diagnostic test kit may comprise:

(a) a known amount of at least one of the metabolites of estrone as described above (or a binding partner) generally bound to a solid phase to form an immunosorbent, or in the alternative, bound to a suitable tag, or plural such end products, etc. (or their binding partners) one of each;
(b) if necessary, other reagents; and
(c) directions for use of said test kit.

In a further variation, the test kit may be prepared and used for the purposes stated above, which operates according to a predetermined protocol (e.g. "competitive", "sandwich", "double antibody", homogeneous enzyme immunoassay, simultaneous multiple analyte immunoassay, etc.), and comprises:

(a) a labeled component which has been obtained by coupling at least one of the metabolites of estrone to a detectable label, said estrone metabolites selected so as to compare the 2-hydroxylation and 16α-hydroxylation pathways of estrone metabolism;

(b) one or more additional immunochemical reagents of which at least one reagent is a ligand or an immobilized ligand, which ligand is selected from the group consisting of:

(i) a ligand capable of bionding with the labeled component (a);
(ii) a ligand capable of binding with a binding partner of the labeled component (a);
(iii) a ligand capable of binding with at least one of the component(s) to be determined; and
(iv) a ligand capable of binding with at least one of the binding partners of at least one of the component(s) to be determined; and

(c) directions for the performance of a protocol for the detection and/or determination of one or more components of an immunochemical reaction between the metabolites of estrone and a specific binding partner thereto.

The following examples set forth the investigations of female and male smokers in an effort to identify a relationship between estrogen metabolism and the pathological states of the test subjects. As a result of the following tests, the relationship between the presence and ratio of the enzymatically hydroxylated estrone products 2-hydroxyestrone and estriol was identified and confirmed. The specific materials and techniques set forth below are exemplary only and may vary, so that the following is presented by way of illustration and not limitation.

## EXAMPLE I

In the present example, a fairly complete profile of estrogen metabolites was examined in smokers and non-smokers, including assays of urinary $16\alpha OHE_1$ and $2OHE_1$, as well as the classical estrogens $E_1$, $E_2$, and $E_3$. The resulting data suggest that catechol estrogens constitute a significantly greater proportion of the total measured urinary estrogens in female smokers compared to non-smokers, and led to the determination of the catechol estrogen index that comprises the quotient of the present invention.

### Materials and Methods

### Subjects

A total of 15 smokers and 14 non-smokers participated in this study; radiometric data from some of these subjects appeared in an earlier report (Michnovicz, J. J., et al., N. ENGL. J. MED., 315:1305-1309 (1986)). All aspects of this study were approved by the Rockefeller University Institutional Review Board. The subjects were healthy, non-obese premenopausal women, ages 21-44, with no history of ongoing menstrual dysfunction, oral contraceptive use, dysthyroidism, or unusual diets. Body-mass index $(weight(kg)/height(m)^2)$ ranged from 19-23, with no significant differences in the means of the two groups. All subjects denied use of prescription or illicit drugs except occasional aspirin or acetaminophen for at least 2 months before the study. Urine samples (30 mL) were obtained between 8:00 and 12:00 AM prior to initiation of radiometric testing (see below), which was conducted within 12 days after the reported onset of menses. Smokers reported consuming 15-30 cigarettes daily, while control subjects reported complete abstention from active cigarette smoking. Cotinine, a major metabolite of nicotine, was undetectable in plasma of non-smokers and ranged between 190 and 400 ng/mL in smokers (assay kindly performed by Dr. Nancy Haley of the American Health Foundation, Valhalla, NY).

### Radioimmunoassays

The radiolabeled 2-hydroxy-[6,7-$^3$H]estrone (sp. act. 45.6 Ci/mmol), [2,4,6,7-$^3$H]estrone (sp. act. 87.5 Ci/mmol), [2,4,6,7-$^3$H]estradiol (sp. act. 97.8 Ci/mmol), and [2,4,6,7-$^3$H]estriol (sp. act. 94.0 Ci/mmol) were purchased from New England Nuclear (Boston, MA). 16-$\alpha$Hydroxy-[6,7-$^3$H]estrone (sp. act. 58 Ci/mmol) was prepared as previously described (S. Ikegawa, et al., STEROIDS 39:557-567 (1979)). Ascorbic acid was obtained from Sigma (St. Louis, MO). Unlabeled $E_1$, $E_3$ and $E_2$ were obtained from Steraloids (Wilton, NH), while unlabeled $16\alpha OHE_1$ and $2OHE_1$ were synthesized by standard techniques. Non-radioactive steroids were recrystallized prior to use. Sep-pak cartridges (Waters Assoc., Milford, MA), Glusulase ($\beta$-glucuronidase and aryl-sulfatase) (Boehringer Mannheim, Indianapolis, IN), and anti-$E_1$ antibodies (Endocrine Sciences, Tarzana, CA) were obtained commercially.

Antibodies to $16\alpha$-$OHE_1$, $E_2$ and $E_3$ were raised as described previously (Fishman, J., et al., SCIENCE, 204:1089-1091 (1979); Ikegawa, S., et al., J. STEROID BIOCHEM., 18:329-332 (1983)), while the anti-$2OHE_1$ antiserum was a gift of Dr. Kanbegawa (Teikyo University, Japan) and was raised against bovine serum albumin linked to 2-hydroxyestrone 17-carboxymethyloxime. Cross-reactivities of the $2OHE_1$ antiserum with $E_1$, $E_2$ and $E_3$, $16\alpha OHE_1$,

$2MeOE_1$, and 2-hydroxyestradiol ($2OHE_2$) were 1.3%, 0.6%, 0.01%, 0.03%, 0.11% and 1.7%, respectively.

Each urine sample (1 or 2 mL) contained approximately 3000 dpm of stably labeled steroid used to determine recovery, and was applied to a $C_{18}$ Sep-pak cartridge followed by elution with 5 mL of methanol. The eluate was dried in vacuo and hydrolyzed with Glusulase for 12 h at 37°C. The hydrolyzed steroids were again extracted on a $C_{18}$ Sep-pak cartridge and eluted with 5 mL of methanol. Aliquots of this final eluate were taken for determination of recovery (averaging 90-95%) and duplicate radioimmunoassay (100 μL). In order to prevent auto-oxidation of catecholestrogens, ascorbic acid (1%) was used in all stages of the assay. Radiolabeled steroids and antisera were incubated with the specimens overnight at 4°C, and radioactivity was measured with a Packard model 3000 scintillation counter. Estrogen levels are expressed relative to urinary creatinine concentration. Statistical analysis was performed with an unpaired t-test. Unless otherwise specified, all values are reported as means ± SEM.

Radiometric Testing

The radiometric method has been described in detail previously (Michnovicz, J. J., et al., N. ENGL. J. MED., 315:1303-1309 (1986); Fishman, J., et al., PROC. NATL. ACAD. SCI. USA, 77:4957-4960 (1980)). Briefly, subjects received 6 μCi of [2-$^3$H]estradiol intravenously (NEN, sp. act. 25.3 Ci/mmol). Blood and urine samples collected over the next 48 h were lyophilized and counted for $^3H_2O$. The extent of the reaction was calculated by dividing the $^3$H specific activity of the total body water volume by the amount of $^3$H in the administered dose.

Results

The amounts of the various urinary estrogen metabolites in female smokers and non-smokers are presented in Table 1 below, along with the extent of 2-hydroxylation as determined by the radiometric technique in each subject. Total estrogen excretion, defined as the sum of the measured urinary metabolites in these random follicular-phase samples, did not differ significantly between smokers and non-smokers (55.3 ± 4.2 vs. 51.7 ± 4.5 μg/g creatinine). Although an attempt was made to collect all urine samples within 12 days after the reported onset of menses, estrogen excretion in a few subjects appeared somewhat higher than the average, suggesting that these individuals were approaching the preovulatory rise in plasma estradiol. Nevertheless, it is apparent that smoking in general exerted little effect on total mid-follicular estrogen excretion. Table 1 demonstrates that the average extent of estradiol 2-hydroxylation measured radiometrically was elevated approximately 50% in smokers (51.6 ± 2.7% vs. 33.3 ± 1.7%).

Table 1

| Comparison of Estrogen Metabolism in Smokers and Non-Smokers | | | |
| --- | --- | --- | --- |
| | Smokers (n=13) | Non-Smokers (n=13) | P Value |
| Extent of 2-Hydroxylation | 52.2 ± 2.6% | 33.6 ± 1.2% | <0.001 |
| $E_2$ | 5.5 ± 0.7 | 3.6 ± 0.3* | <0.02 |
| $E_1$ | 17.5 ± 1.2 | 18.4 ± 2.3 | NS |
| $16\alpha OHE_1$ | 5.6 ± 0.7 | 5.8 ± 0.7 | NS |
| $E_3$ | 10.7 ± 1.1 | 15.6 ± 1.8 | <0.05 |
| $2OHE_1$ | 17.5 ± 2.9 | 8.6 ± 1.0 | <0.01 |
| Total Estrogens | 56.8 ± 5.5 | 52.0 ± 5.8 | NS |
| Values are mean ± SEM. | | | |

* Estrogen metabolites expressed in μg/g creatinine.

Urinary $2OHE_1$ was significantly elevated in smokers, compared with non-smokers (17.2 ± 2.4 vs. 9.4 ± 1.2 μg/g creatinine, P < 0.02). A parallel reduction in urinary $E_3$ was also observed in smokers (10.7 ± 1.1 vs. 15.6 ± 1.8 μg/g, creatinine P<0.05). In contrast, no significant differences were observed in urinary levels of $E_2$, $E_1$ or $16\alpha OHE_1$. As a result of these altered urinary levels, $2OHE_1$ in smokers constituted 31.1% of total measured estrogens, compared with only 18.2% in non-smokers. Increased excretion of $2OHE_1$ thus occurred largely at the expense of decreased excretion of the 16α-hydroxylated metabolite, $E_3$.

Various ratios of 2-hydroxylated to 16α-hydroxylated metabolites were investigated as a means of differentiating the urinary profiles of smokers and non-smokers. A urinary catechol estrogen index, defined by $[2OHE_1]/[E_3]$, was chosen as a ratio of the major metabolite from each pathway.

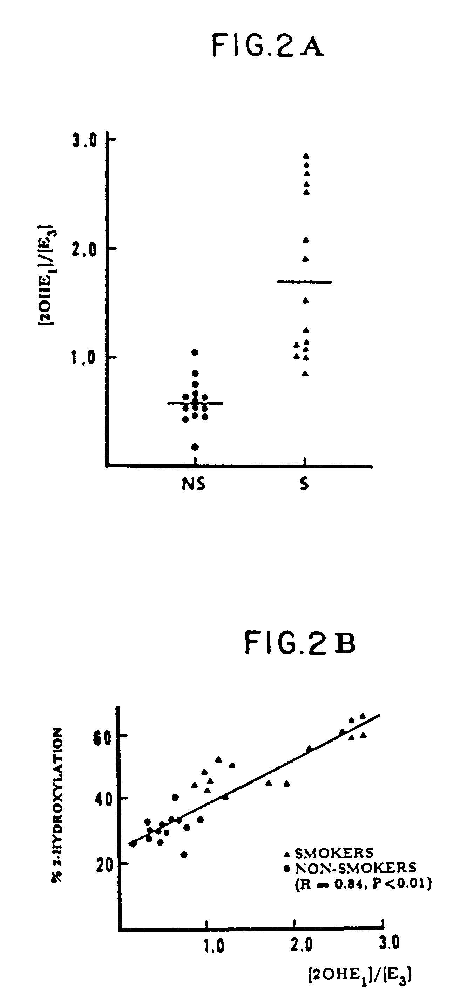

Figure 2A demonstrates the sharp differences in the catechol estrogen index between the two groups. While the values for non-smokers were tightly grouped around the mean value of 0.59 ± 0.08 (range 0.15 to 1.10), the mean for smokers was significantly increased to 1.67 ± 0.21 (range 0.80 to 2.82, P <0.001). Also, there was little overlap between the two groups, and values for several smokers were 3-5 times greater than the mean for non-smokers.

The urinary estrogen ratios were relatively constant throughout the menstrual cycle, despite large variations in estrogen production. For example, in two women from whom urine samples were obtained on each day of a normal menstrual cycle, the means + SEM (n=27 and 30) of various estrogen ratios for each subject were as follows: $[2OHE_1]/[\Sigma All]$ 0.165 ± 0.004 and 0.155 ± 0.004; $[16\alpha OHE_1 + E_3]/[\Sigma All]$ 0.391 ± 0.012 and 0.485 ± 0.007 $[2OHE_1]/[E_3]$ 0.689 ± 0.034 and 0.433 ± 0.014.

Discussion

The above data demonstrate in premenopausal women that mid-follicular urinary estrogen excretion patterns of smokers differ significantly from those of non-smokers. The principal alteration involves increased $2OHE_1$ excretion and concomitantly decreased $E_3$ excretion among smokers, with no significant difference in the total of these five measured estrogens. These data are in agreement with the earlier finding of increased estrogen 2-hydroxylation measured radiometrically in female smokers (Michnovicz, J. J., et al., N. ENGL. J. MED., 315:1305-1309 (1986)).

MacMahon et al. (N. ENGL. J. MED., 307:1062-1065 (1982)) previously reported that cigarette smokers had decreased urinary excretion of $E_2$, $E_1$ and $E_3$ during the luteal but not the follicular phase of the menstrual cycle. They concluded that smokers had decreased estrogen production in the luteal phase. The present study did not detect a significant difference in total urinary estrogen excretion in the follicular phase. However, the shift toward catechol estrogen excretion in smokers indicates that the exclusion of $2OHE_1$ from the previous analysis precluded an accurate estimate of total estrogen production. The present data show that among the classical estrogens only $E_3$ is reduced in the follicular phase of smokers. Since the relative ratios of the various metabolites do not change appreciably during the menstrual cycle, the difference should presumably be observed in both the follicular and luteal phases.

The discovery and analysis of the urinary catechol estrogen index, $[2OHE_1]/[E_3]$, suggests that randomly collected urine samples may provide a reliable estimate of increased 2-hydroxylation in selected populations. Urinary assays would obviate the need to perform in vivo radiometric testing when comparing 2- and 16αhydroxylation in populations suspected of differing in estrogen metabolism. Although urinary estrogen ratios such as $[2OHE_1]/[E_3]$ are sensitive to inter- and intra-assay variation (typically 5 to 10%) (Fishman, J., et al., SCIENCE, 204:1089-1091 (1979)), significant differences in the means of well-defined populations should still be apparent, as they are in these premenopausal female smokers compared with matched non-smokers. Combined radiometric and urinary assays in men have also demonstrated the usefulness of the catechol estrogen index (Michnovicz, J. J., et al. METABOLISM 38:537-541 (1989).

The above experiment was unable to measure urinary $2MeOE_1$, another potentially important metabolite in the 2-hydroxylative pathway (see Figure 1). Nevertheless, published data suggest that the relative amounts of this metabolite in the urine of premenopausal females are only about 10-15% of $2OHE_1$ (Adlercreutz, H., et al. J. STEROID BIOCHEM., 24:289-296 (1986)). Thus, changes in the excretion of this metabolite in smokers are unlikely to affect the validity of the urinary catechol estrogen index, defined solely by $[2OHE_1]/[E_3]$, in detecting differences in metabolism among selected populations.

Although both smoking and obesity possibly affect estrogen production, the metabolic changes outlined above may also modify hormone-dependent disease in humans. In addition to smoking and altered body weight, other factors influencing the extent of 2-hydroxylation in humans include short-term (Anderson, K. E., et al., J. CLIN. ENDOCRINOL. METAB., 59:103-107 (1984)) and long-term (Adlercreutz, H., et al., J. STEROID BIOCHEM., 24:289-295 (1986)) dietary changes, dysthyroidism (Fishman, J., et al., J. CLIN. ENDOCRINOL. METAB., 25:365-368 (1964)), and liver disease (Zumoff, B., et al., J. CLIN. INVEST., 47:20-25 (1968)). The present studies and the resulting identification of the quotient or index of the invention shed light on the control of estrogen metabolism and should be useful in formulating safer therapeutic approaches toward reducing risk for estrogen-related disease.

EXAMPLE II

In this series of experiments, groups of male smokers and non-smokers were examined by analysis of the urinary estrogen catechol index of the present invention in conjunction with in vivo radiometric analysis for purposes of corroboration. The procedures and results of the experiments are set forth below.

Materials and Methods

Subjects

All of the subjects (n=36) were healthy, non-obese males, 19 to 57 years of age, using no medications except occasional aspirin or acetaminophen. All procedures were approved by the Rockefeller University Institutional Review Board and were performed after obtaining written informed consent from the participant. Physical examination and routine blood tests were normal for each subject. There were no significant differences in the mean age, weight, height or body mass index between the smokers and nonsmokers (Table 2). Nonsmokers denied active smoking, and smokers were included only if they reported the daily inhalation of at least 15 cigarettes (mean 24 ± 4, range 15 to 55).

Table 2

| Clinical Data of 20 Smokers and 16 Nonsmoking Controls | | | | |
|---|---|---|---|---|
| Subjects | Age | Height | Weight | BMI* |
| | (1 yr) | (m) | (kg) | $(kg/m_2)$ |
| Smokers | 32.6 ± 2.6 | 1.78 ± 0.02 | 72.2 ± 1.7 | 22.8 ± 0.3 |
| | (19-57) | (1.60-1.91) | (55.5-85.7) | (19.7-25.3) |
| Nonsmokers | 28.6 ± 1.2 | 1.78 ± 0.02 | 75.4 ± 2.3 | 23.7 ± 0.4 |
| | (23-40) | (1.63-1.92) | (60.0-90.8) | (20.2-26.8) |

To measure estradiol 2-hydroxylation radiometrically (Fishman, J., et al., PROC. NATL. ACAD. SCI. USA, 77: 4957-4960 (1980); Michnovicz, J. J., et al., N. ENGL. J. MED., 315:1305-1209 (1986)), each subject received 6 µCi of [2-$^3$H]-estradiol intravenously (New England Nuclear Corporation, Boston; specific activity 25.3 Ci/mmol). Blood and urine samples collected over the next 48 hours were lyophilized and counted for $^3H_2O$. The extent of the reaction for each subject was calculated by dividing the total $^3$H present in the body water volume by the $^3$H administered as [2-$^3$H]-estradiol. The body water volume was estimated using a bioelectrical impedance device (Model BIA-103, RJL Systems, Detroit).

Cotinine, a major metabolite of nicotine and a biochemical marker of tobacco consumption, was measured by radioimmunoassay (Haley, N. J., AM. J. PUBLIC HEALTH, 73:1204-1207 (1983)). With an assay sensitivity of 0.4 ng/mL, cotinine was undetectable in the serum of all nonsmokers, and ranged from 89 to 487 ng/mL of serum in the smokers.

Random urine samples (30 mL) obtained between 8 AM and 12 noon prior to the radiometric test were stored for future analysis at -60°C in the presence of ascorbic acid (1%). Frozen urine samples were not available for assay in three of the subjects (two smokers and one nonsmoker). Radioimmunoassays of urinary estrogen metabolites were performed following enzymatic hydrolysis of conjugates, as previously described (Michnovicz, J. J., et al., STEROIDS, 52 (1988)). The interassay coefficients of variability for the urinary estrogen assays varied between 5.0% and 10.0%. Statistical significance was determined using Student's t test, and all values are reported as mean ± SEM unless otherwise specified.

Results

The results of the radiometric assay in smokers and nonsmokers are shown in Fig. 3A. The average extent of estradiol 2-hydroxylation for 16 nonsmokers was 24.6% ± 1.9% of the administered dose. For the 20 smokers, the extent of reaction averaged 43.4 ± 1.9%, an increase of approximately 70% (P < .001). Cotinine levels in the smokers averaged 242 ± 22 ng/mL (range 89 to 487 ng/mL). Individual cotinine values did not correlate well with the extent of 2-hydroxylation in this group of heavy smokers (P=.61).

The data shown in Fig. 3A indicate that values for 2-hydroxylation in many smokers overlapped with those of the nonsmoking population. Some lean individuals smoking over two packs per day, and thus expected to show a higher extent of reaction (Fishman, J., et al., CLIN. PHARMACOL. THER., 22:721-728 (1977)), were sometimes still within the normal range. This suggests the presence of other variables, which might counteract the inducing effects of tobacco. Values from men who reported substantial ethanol use (habitual consumption on weekends of at least 12 cans of beer or its equivalent in hard liquor) are indicated in Fig. 3A with a circle. The values for four of five such individuals fell below their respective means.

Urinary estrogen metabolites were measured to determine whether the radiometric data were reflected in endogenous estrogen excretion patterns. Values of urinary estrogen metabolites in both populations are summarized in Table 2. A significant elevation in the excretion of $2OHE_1$ was found in the smokers (10.4 ± 1.3 µg/g creatinine v 6.3 ± 0.7, P = .011), as well as a trend toward reduced $E_3$, consistent with the metabolic shift measured radiometrically.

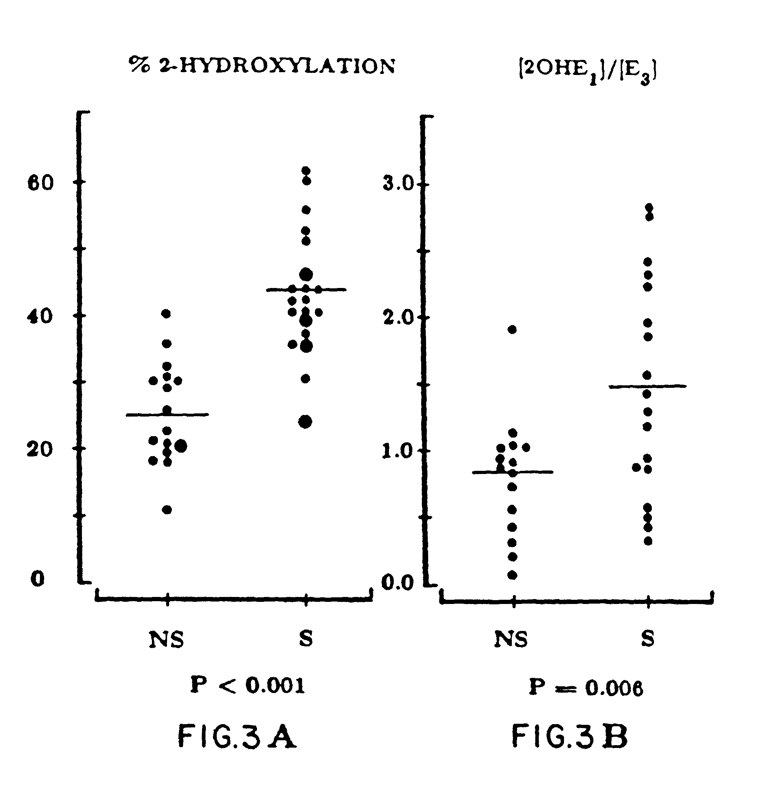

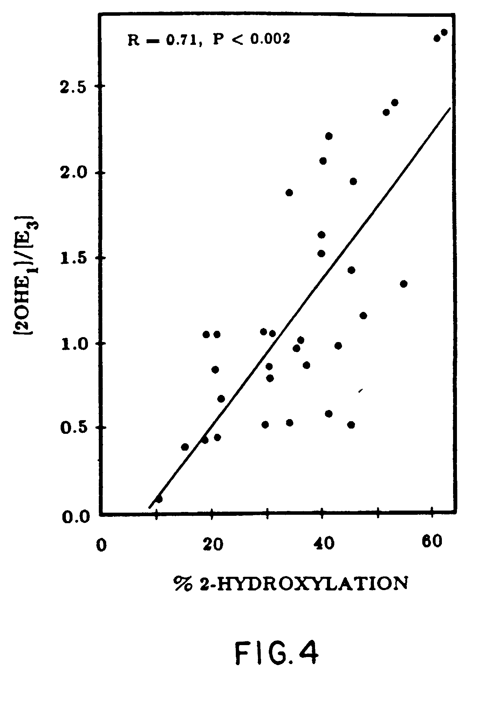

A urinary catechol estrogen index defined by $[2OHE_1]/[E_3]$, previously shown to correlate with the extent of 2-hydroxylation developed in accordance with the present invention, was calculated in female smokers. This index minimizes the effect of variations in the absolute amount of estrogen present in a random urine sample (Michnovicz, J. J., et al., STEROIDS, 52 (1988)). Results are shown for both groups in Fig. 3B. The average for the smokers (1.46 ± 0.19) was significantly greater than that of the nonsmokers (0.81 ± 0.11, P = .006). More variation in this ratio was found among the smokers, as well as considerable overlap with the values of nonsmokers. A strong correlation was found between the urinary catechol estrogen index and the radiometric extent of reaction from each subject (r = 0.71, P = .001; Fig. 4).

Table 3

| Summary of Urinary Estrogen Metabolites | | | |
|---|---|---|---|
| | Smokers | Nonsmokers | P |
| $E_2$ | 3.9 ± 0.4 | 3.4 ± 0.3 | NS |
| $E_1$ | 9.6 ± 0.8 | 9.7 ± 1.0 | NS |
| $16\alpha OHE_1$ | 3.3 ± 0.5 | 2.5 ± 0.2 | NS |
| $E_3$ | 7.9 ± 0.8 | 9.0 ± 0.9 | NS |
| $2OHE_1$ | 10.4 ± 1.3 | 6.3 ± 0.7 | .011 |
| Total estrogens* | 35.2 ± 2.7 | 30.9 ± 1.9 | NS |
| $[2OHE_1]/[E_3]$ | 1.46 ± 0.19 | 0.81 ± 0.11 | .006 |
| Estrogen metabolites (mean ± SEM) are expressed as µg/g creatinine. | | | |

\* This represents the sum of $E_1$, $E_2$, $E_3$, $16\alpha OHE_1$, and $2OHE_1$.

Discussion

The radiometric data reported here demonstrate that induction of estradiol 2-hydroxylation is associated with moderately heavy tobacco use in men. Urinary excretion of endogenous 2-hydroxyestrone is significantly increased in male smokers, both in absolute amount and relative to estriol, suggesting that the enhanced 2-hydroxylation reduces the bioavailability of the active estrogen metabolites 16α-hydroxyestrone and estriol. Because 2-hydroxyestrogens are weak agonists (Martucci, C. P., et al., ENDOCRINOLOGY, 105:1288-1292 (1979)), their increased formation is likely to reduce the overall estrogenic stimulation in the liver, the predominant site of 2-hydroxylation (Barbieri, R. L., et al., STEROIDS, 32:529-538 (1978).

In addition to identifying the anti-estrogen effects of smoking in both males and females and postulating, as the underlying physiological and biochemical bases and implications of these effects, the above results further confirm that the quotient determined in accordance with the invention provides consistent information regarding pathological status that is valid for both males and females and is independent of physiological fluctuations.

EXAMPLE III

This example is taken from a study performed by Galbraith et al. and identified herein as Reference (3) respecting the activity of the histamine $H_2$-receptor antagonist Cimetidine, which has been widely used to treat peptic ulceration. The study performed by Galbraith et al. was motivated by the prior noted activity of Cimetidine to cause gynecomastia and sexual dysfunction in certain men, in combination with demonstrated activity as an inhibitor of the cytochrome P-450-dependent biotransformation of certain drugs. The investigators accordingly sought to determine whether Cimetidine also might inhibit the cytochrome P-450-dependent metabolism of estradiol.

The study comprised the radiometric analysis of urine and serum samples taken from nine normal male volunteers who had received twice daily oral doses of 800 mg per dose of Cimetidine for a period of two weeks. The study showed that, during this time, the 16α-hydroxylation of estradiol was unaffected; however, the urinary excretion of 2-hydroxyestrone decreased by approximately 25% (P<0.0002), and the serum concentration of estradiol increased by approximately 20% (P<0.04).

A review of this study and, in particular, Table 2 of the study appearing on page 271 thereof, illustrates the predictive value of the index determined from the ratio of $2OHE_1/E_3$. Table 2 is reproduced herein below as Table 4.

Table 4

| Effect of Cimetidine on the Urinary Excretion of Estrogens* | | | |
|---|---|---|---|
| | Before Cimetidine | After Cimetidine | P Value** |
| | nmol/nmol of creatinine | | |
| Estrone | $3.80 \pm 0.34$ | $3.32 \pm 0.26$ | 0.03 |
| Estradiol | $1.28 \pm 0.15$ | $1.25 \pm 0.10$ | NS |
| $16\alpha$-Hydroxyestrone | $1.08 \pm 0.11$ | $1.05 \pm 0.08$ | NS |
| 2-Hydroxyestrone | $1.71 \pm 0.20$ | $1.27 \pm 0.19$ | 0.0002 |
| Total estrogens*** | $10.7 \pm 1.1$ | $9.9 \pm 0.9$ | 0.02 |
| 2-Hydroxyestrone/estriol | $0.74 \pm 0.18$ | $0.47 \pm 0.10$ | 0.015 |

* Timed overnight urine samples were collected and assayed, as described in Methods, before and after treatment with cimetidine (800 mg orally twice a day for 14 days. Results are expressed as means ± SEM. The number of measurements was nine in each case.

** The P values were calculated with the use of paired Student t-tests, NS denotes not significant.

*** Total estrogens comprises estrone, estradiol, estriol, $16\alpha$-hydroxyestrone, and 2-hydroxyestrone.

As noted in the Galbraith et al. article, the ratio of 2-hydroxyestrone to estriol was reduced by approximately 40% after Cimetidine treatment. The authors go on to state that Cimetidine appears to possess therapeutic value in the inhibition of estrogen-dependent disease.

The above data are presented herein to substantiate that the index and quotient of the present invention possess predictive value with respect to pathology that goes beyond the scope and instances set forth in Examples 1 and 2.

EXAMPLE IV

This experiment is taken from a publication by Michnovicz and Galbraith listed herein as Reference (4). In this instance, the authors examined the effect of a brief period of hyperthyroidism on estradiol hydroxylation at both the C-2 and C-$16\alpha$ sites. An in vivo radiometric assay with healthy male volunteers was employed, and a two-week course of thyroxine (4.3 mcg/kg/d) was administered. While the primary thrust of this study revealed that the C-2 pathway may be regulated whereas the C-$16\alpha$ pathway appears to be refractory, the data collected and analyzed in Table 1 of the publication at page 23 thereof, which table is reproduced as Table 5 hereinbelow, illustrated again in cumulative fashion the validity and possible value of the index of $2OHE_1/E_3$.

Table 5

| Effect of Thyroxine in Urinary Estrogens[a] | | | |
|---|---|---|---|
| Metabolite | Pre[b] | Post[c] | P Value |
| $E_2$ | $2.78 \pm 0.23$ | $3.03 \pm 0.27$ | NS |
| $E_1$ | $7.36 \pm 0.39$ | $7.30 \pm 0.74$ | NS |
| $16\alpha OHE_1$ | $2.07 \pm 0.24$ | $2.24 \pm 0.17$ | NS |
| $E_3$ | $5.84 \pm 0.88$ | $5.37 \pm 0.78$ | NS |
| $2OHE_1$ | $2.88 \pm 0.32$ | $5.30 \pm 0.85$ | 0.01 |
| Total estrogens | $20.93 \pm 1.58$ | $23.24 \pm 2.41.$ | NS |
| $[2OHE_1]/[E_3]$ | $0.56 \pm 0.09$ | $1.05 \pm 0.14$ | 0.01 |

[a] Estrogen metabolites (means ± SEM) are expressed as μg creatinine.

[b] Pre: baseline study

[c] Post: following two weeks of thyroxine.1.5

As can be seen from the table, a significant reduction in the index was noted, and it was found that urinary 2-hydroxyestrone was significantly greater after thyroxine administration and that the index nearly doubled, mostly due to the elevated excretion of $2OHE_1$. Clearly, the condition of hyperthyroidism would be demonstrably reflected in the elevation of the index of the present invention, so that the index would serve to accurately predict this particular pathology. Accordingly, the data presented in Michnovicz et al. is further corroborative of the scope and value of the index of the present invention.

EXAMPLE V

In this study by two of the inventors herein, which study was just recently published, certain dietary indoles were examined for their induction of hepatic estradiol 2-hydroxylation. In particular, indole-3-carbinol (I3C) was administered to humans at a rate of 500 mg daily for one week. The results of this study reveal that I3C significantly increased the extent of estradiol 2-hydroxylation and indicated that I3C strongly influences estradiol metabolism in humans.

In conjunction with this study, certain comparative data were developed between the administration of I3C to women and men, and the administration of a high fiber diet to women. The comparative results are presented in Table 6A, B and C below, wherein Table 6A represents the data from women taking I3C, Table 6B represents the data respecting the women who were placed on a high fiber diet, and Table 6C represents the data gathered from the sample of men who were administered I3C.

Table 6A

| Women Taking 13C (N=5) | | | |
|---|---|---|---|
| Metabolite | Pre | Post | P Value |
| $[2OHE_1]/[E_1]$ | $1.15 \pm 0.24$ | $1.53 \pm 0.20$ | <0.05 |
| $[E_3]/[E_1]$ | $1.04 \pm 0.20$ | $0.74 \pm 0.11$ | NS |
| $[2OHE_1]/[E_3]$ | $1.27 \pm 0.30$ | $2.35 \pm 0.50$ | <0.005 |

Table 6B

| Women Going on High Fiber Diet (N=6) | | | |
|---|---|---|---|
| Metabolite | Pre | Post | P Value |
| $[E_3]$ | $9.3 \pm 2.8$ | $8.2 \pm 3.2$ | NS |
| $[E_1]$ | $10.4 \pm 2.7$ | $10.8 \pm 2.7$ | NS |
| $[2OHE_1]$ | $11.5 \pm 3.2$ | $12.8 \pm 4.1$ | NS |
| $[2OHE_1]/[E_3]$ | $1.32 \pm 0.35$ | $1.63 \pm 0.40$ | <0.05 |

Table 6C

| Men Taking I3C (N=7) | | | |
|---|---|---|---|
| Metabolite | Pre | Post | P Value |
| $[E_1]$ | $8.4 \pm 1.8$ | $8.9 \pm 2.3$ | NS |
| $[E_3]$ | $6.4 \pm 1.8$ | $4.2 \pm 1.3$ | <0.006 |
| $[2OHE_1]$ | $6.6 \pm 2.3$ | $8.2 \pm 3.5$ | NS |
| $[2OHE_1]/[E_3]$ | $1.06 \pm 0.11$ | $2.04 \pm 0.35$ | <0.02 |

The study by Michnovicz et al. as to indole-3-carbinol confirmed earlier data with respect to the therapeutic and prophylactic value of ingestion of foods containing this compound. In this respect, both I3C and the high fiber diet as illustrated in the above unpublished data presented in Tables 6A-C above, confirmed that I3C and high fiber are potent inducers of estradiol 2-hydroxylation in similar fashion to the effect evidenced by smoking, and as presented in Example 1 herein. The data presented in Tables 6A-C include comparisons of the C-2 pathway index, which is likewise confirmatory of this therapeutic effect. Thus, the data presented as to the index in Tables 6A-C are cumulative and confirmatory of the predictive diagnostic value of the index of the present invention and its corresponding therapeutic relevance.

Clearly, the components of the index may be monitored and may correspondingly offer data to the clinician respecting the pathological state of patients who are being administered a potential therapeutic agent such as I3C or drugs or agents that may mimic the activity of I3C or other therapeutic agents having effect on estrogen metabolism as part of their activity profile.

**Claims**

1. A method for detecting pathology in mammals which comprises:

   A. isolating at least two distinct metabolites of estrone from a biological sample taken from the mammal under examination, said metabolites selected so as to compare the 2-hydroxylation and 16α-hydroxylation pathways of estrone metabolism;
   B. determining the quantity of each of said metabolites in said sample;
   C. correlating the ratio of the quantities of each of said metabolites with each other to arrive at a ratio of said metabolites; and
   D. comparing said ratio with an extrinsic ratio derived either previously from the mammal under testing, or from the testing of other subjects of the same species, to detect any alterations in said estrogen metabolism, said alterations identifying the existence of said pathology.

2. The method of Claim 1 wherein said metabolites of estrone are selected from the group consisting of products of enzymatic hydroxylation of estrone products of enzymatic reduction of estrone, products of enzymatic oxidation of estrone, and products of enzymatic methylation of estrone.

3. The method of Claim 1 wherein said products are selected from the group consisting of 2-hydroxyestrone, 4-hydroxyestrone, 15α-hydroxyestrone, 16α-hydroxyestrone, 2-methoxyestrone, estriol, 17β-estriol, epiestriol, 16-ketoestradiol and 15α-hydroxyestradiol.

4. The method of Claim 1 wherein said products comprise 2-hydroxyestrone and estriol.

5. The method of anyone of Claims 1 to 4, wherein said concentration is measured by chromatographic analysis.

6. The method of Claim anyone of Claims 1 to 4, wherein said concentration is measured by immunochemical analysis.

7. The method of Claim anyone of Claims 1 to 4, wherein said concentration is measured by radiometric analysis.

8. The method of Claim anyone of Claims 1 to 4, wherein said concentration is measured by receptor assay.

9. The method of Claim 1 wherein said pathology is selected from osteoporosis, endometriosis, obesity, atherosclerosis, endometrial cancer, breast cancer, heart disease, infertility, gastrointestinal disease, central nervous system disease, and combinations thereof.

10. A method for measuring the presence of metabolites of estrone in a mammal in which alteration of estrogen metabolism is implicated, said metabolites selected so as to compare the 2-hydroxylation and 16α-hydroxylation pathways of estrone metabolism, wherein each metabolite is measured by:

    A. preparing at least one antibody which specifically binds to an estrone metabolite;
    B. placing a detectable label on either said metabolite or said antibody;
    C. immobilizing the unlabelled material from Step B on a solid support;
    D. contacting the labelled material from Step B and the immobilized material of step C with a biological sample from a mammal in which said estrogen metabolism alteration is suspected;
    E. separating the labelled material from Step D that is bound to said immobilized material from material from Step D not bound to said immobilized material; and
    F. examining said bound material for the presence of said labelled material to determine by reference to a standard the quantity of each of said estrone metabolites in the sample, correlating the quantities of each of said metabolites with each other to arrive at a ratio of said metabolites, and comparing said ratio with an extrinsic ratio derived either previously from the mammal under test, or from testing of other subjects of the same species, to determine any alteration in said estrogen metabolism and to detect the existence of a pathology therefrom.

11. A method for measuring the presence of the metabolites of estrone in a mammal in which alteration of estrone metabolism is implicated in order to determine said alteration, said metabolites selected so as to compare the 2-hydroxylation or 16α-hydroxylation pathways of estrone metabolism, wherein each of said metabolites is measured by:

A. preparing at least one corresponding antibody which specifically binds to an estrone metabolite;

B. placing a detectable label on either said metabolite, or said antibody of Step A;

C. placing the labelled material from Step B and the corresponding antibody, or metabolite that is not labelled, in contact with a biological sample from a mammal in which said estrogen metabolism alteration is suspected to form a unitary mixture; and

D. examining the mixture of Step C for the presence of activity of said labelled material to determine by reference to a standard the quantity of each of said estrone metabolites in the sample, correlating the quantities of each of said metabolites with each other to arrive at a ratio of said metabolites, and comparing said ratio with an extrinsic ratio derived either previously from the mammal under test, or from testing of other subjects of the same species, to determine any alteration in said estrogen metabolism and to detect the existence of a pathology therefrom.

12. The method of Claims 10 or 11, wherein the label is an enzyme.

13. The method of Claim 12, wherein the label is selected from the group consisting of peroxidase, β-glucuronidase, β-D-glucosidase, β-D-galactosidase, urease, glucose oxidase plus peroxidase, galactose oxidase plus peroxidase, hexokinase plus GPDase, glucose oxidase plus alkaline phosphatase, NAD oxidoreductase plus luciferase, phosphofructokinase plus phosphoenol pyruvate carboxylase, aspartate aminotransferase plus phosphoenol pyruvate decarboxylase, and alkaline phosphatase.

14. The method of Claims 10 or 11, wherein the label is a chemical which fluoresces.

15. The method of Claim 14 wherein the chemical is selected from the group consisting of fluorescein, rhodamine, and auramine.

16. The method of Claims 10 or 11 wherein the label is a radioactive element.

17. The method of Claim 16 wherein the radioactive element is selected from the group consisting of $^{14}C$, $^{125}I$, $^{131}I$, $^{35}S$, $^{57}Co$, $^{59}Fe$ and $^{3}H$.

18. A test kit for the detection of a pathology in which an alteration in estrogen metabolism is implicated, using the serum, tissue or aqueous medium of a mammal under test, comprising:

A. a predetermined amount of at least one labelled immunochemically reactive component obtained by the direct or indirect attachment of one or more estrone metabolites, or a specific binding partner thereto, to a detectable label, said estrone metabolites selected so as to compare the 2-hydroxylation and 16α-hydroxylation pathways of estrone metabolism;

B. other reagents; and

C. directions for use of said kit.

19. The test kit of Claim 18, wherein the label is an enzyme.

20. The test kit of Claim 19, wherein the label is selected from the group consisting of peroxidase, β-glucuronidase, β-D-glucosidase, β-D-galactosidase, urease, glucose oxidase plus peroxidase, galactose oxidase plus peroxidase, hexokinase plus GPDase, glucose oxidase plus alkaline phosphatase, NAD oxidoreductase plus luciferase, phosphofructokinase plus phosphoenol pyruvate carboxylase, aspartate aminotransferase plus phosphoenol pyruvate decarboxylase, and alkaline phosphatase.

21. The test kit of Claim 18, wherein the label is a chemical which fluoresces.

22. The test kit of Claim 21, wherein the chemical is selected from the group consisting of fluorescein, rhodamine, and auramine.

23. The test kit of Claim 18, wherein the label is a radioactive element.

24. The test kit of Claim 23, wherein the radioactive element is selected from the group consisting

of $^{14}C$, $^{125}I$, $^{131}I$, $^{35}S$, $^{57}Co$, $^{59}Fe$ and $^{3}H$.

25. An <u>in vitro</u> method for monitoring the course and efficacy of a drug or dietary therapy in which an alteration in estrogen metabolism is implicated comprising performing the method of Claim 1 to periodically monitor the ratio to note any changes.

**Patentansprüche**

1. Verfahren zum Nachweis von Krankeitszuständen in Säugetieren mit folgenden Schritten:

A) Isolieren von wenigstens zwei verschiedenen Metaboliten von Östron aus einer biologischen Probe, die von dem untersuchten Säugetier genommen ist, wobei die Metaboliten ausgewählt sind zum Vergleichen der 2-Hydroxylierungs- und 16α-Hydroxylierungswege des Östron-Metabolismus;
B) Bestimmen der Menge jedes dieser Metabolite in der Probe;
C) in Beziehung Setzen des Verhältnisses der Mengen jeder der Metabolite zueinander zur Ermittlung eines Verhältnisses der Metabolite und
D) Vergleichen des Verhältnisses mit einem äußeren Verhältnis, das entweder zuvor von dem untersuchten Säugetier oder von der Untersuchung anderer Subjekte der gleichen Spezies gewonnen wurde, um alle Abweichungen im Östrogen-Metabolismus nachzuweisen, welche das Vorhandensein der Krankheitszustände aufzeigen.

2. Verfahren nach Anspruch 1, worin die Östron-Metabolite ausgewählt sind aus der Gruppe von Produkten der enzymatischen Hydroxylierung von Östron, Produkten der enzymatischen Reduktion von Östron, Produkten der enzymatischen Oxidation von Östron und Produkten der enzymatischen Methylierung von Östron.

3. Verfahren nach Anspruch 1, worin die Produkte ausgewählt sind aus der Gruppe 2-Hydroxyöstron, 4-Hydroxyöstron, 15α-Hydroxyöstron, 16α-Hydroxyöstron, 2-Methoxyöstron, Östriol, 17β-Östriol, Epiöstriol, 16-Ketoöstradiol und 15α-Hydroxyöstradiol.

4. Verfahren nach Anspruch 1, wobei zu den Produkten 2-Hydroxyöstron und Östriol gehören.

5. Verfahren nach einem der Ansprüche 1 bis 4, worin die Konzentration durch chromatographische Analyse gemessen wird.

6. Verfahren nach einem der Ansprüche 1 bis 4, worin die Konzentration durch immunochemische Analyse gemessen wird.

7. Verfahren nach einem der Ansprüche 1 bis 4, worin die Konzentration durch radiometrische Analyse gemessen wird.

8. Verfahren nach einem der Ansprüche 1 bis 4, worin die Konzentration durch RezeptorUntersuchung gemessen wird.

9. Verfahren nach Anspruch 1, worin der Krankheitszustand ausgewählt ist aus Osteoporose, Endometriose, Übergewicht, Arteriosklerose, endometrisches Carcinom, Brustcarcinom, Herzkrankheit, Unfruchtbarkeit, Krankheit des Verdauungstraktes, Krankheit des zentralen Nervensystems und Kombination derselben.

10. Verfahren zur Messung der Anwesenheit von Metaboliten von Östron in einem Säugetier bei einer Veränderung des Östrogen-Metabolismus, wobei die Metaboliten ausgewählt sind zum Vergleichen der 2-Hydroxylierungs- und 16α-Hydroxylierungswege des Östron-Metabolismus, wobei jeder Metabolit gemessen wird durch folgende Schritte:

A) Herstellen wenigstens eines Antikörpers, der spezifisch an einen Östron-Metaboliten bindet;
B) Anbringen einer detektierbaren Markierung an entweder dem Metaboliten oder dem Antikörper;
C) Immobilisieren des unmarkierten Materials des Schritts B) an einem festen Träger;
D) in Berührung Bringen des markierten Materials des Schritts B) und des immobilisierten Materials des Schritts C) mit einer biologischen Probe von einem Säugetier, in welchem die Veränderung des Östrogen-Metabolismus vermutet wird;

E) Abtrennen des markierten Materials der Stufe D), das an das immobilisierte Material gebunden ist, vom Material der Stufe D), das an das immobilisierte Material nicht gebunden ist;

F) Untersuchung des gebundenen Materials auf die Anwesenheit des markierten Materials, um durch Bezug auf einen Standard die Menge jedes der Östron-Metabolite in der Probe zu bestimmen, Korrelieren der Mengen jedes der Metabolite miteinander, um ein Verhältnis der Metabolite zu gewinnen, und Vergleichen des Verhältnisses mit einem äußeren Verhältnis, das entweder zuvor von dem untersuchten Säugetier oder vom Testen anderer Subjekte der gleichen Spezies abgeleitet wurde, um jede Veränderung im Östrogen-Metabolismus zu bestimmen und daraus das Vorhandensein eines Krankheitszustandes nachzuweisen.

11. Verfahren zum Messen der Anwesenheit von Östron-Metaboliten in einem Säugetier, in welchem eine Veränderung des Östron-Metabolismus anzunehmen ist, um diese Veränderung zu bestimmen, wobei die Metaboliten ausgewählt sind zum Vergleichen der 2-Hydroxylierung oder $16\alpha$-Hydroxylierungswege des Östron-Metabolismus, und worin jeder der Metabolite gemessen wird durch folgende Stufen:

A) Herstellen wenigstens eines entsprechenden Antikörpers, der spezifisch an einen Östron-Metaboliten bindet;

B) Anbringen einer detektierbaren Markierung an entweder dem Metaboliten oder dem Antikörper der Stufe A);

C) Kontaktieren des markierten Materials der Stufe B) und des entsprechenden Antikörpers oder des nichtmarkierten Metaboliten mit einer biologischen Probe eines Säugetiers, worin die Veränderung des Östrogen-Metabolismus vermutet wird, um eine einheitliche Mischung zu bilden, und

D) Untersuchung der Mischung der Stufe C) auf das Vorhandensein von Aktivität des markierten Materials, um durch Bezug auf einen Standard die Menge jedes der Östron-Metaboliten in der Probe zu bestimmen, Korrelieren der Mengen jedes der Metabolite miteinander, um ein Verhältnis der Metabolite zu gewinnen, und Vergleichen dieses Verhältnisses mit einem äußeren Verhältnis, das entweder zuvor von dem untersuchten Säugetier oder vom Testen anderer Subjekte der gleichen Spezies abgeleitet wurde, um jede Veränderung im Östrogen-Metabolismus zu bestimmen und daraus das Vorhandensein eines Krankheitszustandes nachzuweisen.

12. Verfahren nach Anspruch 10 oder 11, worin die Markierung ein Enzym ist.

13. Verfahren nach Anspruch 12, worin die Markierung ausgewählt ist aus der Gruppe von Peroxidase, $\beta$-Glucuronidase, $\beta$-D-Glucosidase, $\beta$-D-Galactosidase, Urease, Glucoseoxidase plus Peroxidase, Galaktoseoxidase plus Peroxidase, Hexokinase plus GPDase, Glukoseoxidase plus alkalische Phosphatase, NADoxireduktase plus Luciferase, Phosphorfruktokinase plus Phosphoenolpyruvatcarboxylase, Aspartataminotransferase plus Phosphoenolpyruvatdecarboxylase, und alkalische Phosphatase.

14. Verfahren nach Anspruch 10 oder 11, worin die Markierung ein fluoreszierender chemischer Stoff ist.

15. Verfahren nach Anspruch 14, worin der chemische Stoff ausgewählt ist aus der Gruppe Fluorescein, Rhodamin und Auramin.

16. Verfahren nach Anspruch 10 oder 11, worin die Markierung ein radioaktives Element ist.

17. Verfahren nach Anspruch 16, worin das radioaktive Element ausgewählt ist aus der Gruppe $^{14}$C, $^{125}$I, $^{131}$I, $^{35}$S, $^{57}$Co, $^{59}$Fe und $^{3}$H.

18. Testpackung zum Nachweis eines Krankheitszustandes, bei dem eine Veränderung im Östrogen-Metabolismus auftritt, unter Verwendung von Serum, Gewebe oder wäßrigem Medium eines untersuchten Säugetiers mit folgendem Inhalt:

A) eine vorbestimmte Menge von wenigstens einer markierten immunochemisch reaktiven Komponenten, die durch die direkte oder indirekte Bindung von einem oder mehreren Östron-Metaboliten oder einem spezifischen Bindungspartner an diese an eine detektierbare Markierung erhalten wurde, wobei die Östron-Metabolite ausgewählt sind zum Vergleichen der 2-Hydroxylierungs- und $16\alpha$-Hydroxylierungswege des Östron-Metabolismus;

B) andere Reagenzien und

C) Gebrauchsanweisungen für die Packung.

**19.** Testpackung nach Anspruch 18, worin die Markierung ein Enzym ist.

**20.** Testpackung nach Anspruch 19, worin die Markierung ausgewählt ist aus der Gruppe Peroxidase, β-Glucuronidase, β-D-Glucosidase, β-D-Galactosidase, Urease, Glucoseoxidase plus Peroxidase, Galaktoseoxidase plus Peroxidase, Hexokinase plus GPDase, Glukoseoxidase plus alkalische Phosphatase, NADoxireduktase plus Luciferase, Phosphorfruktokinase plus Phosphoenolpyruvatcarboxylase, Aspartataminotransferase plus Phosphoenolpyruvatdecarboxylase, und alkalische Phosphatase.

**21.** Testpackung nach Anspruch 18, worin die Markierung ein fluoreszierender chemischer Stoff ist.

**22.** Testpackung nach Anspruch 21, worin der chemische Stoff ausgewählt ist aus der Gruppe Fluorescein, Rhodamin und Auramin.

**23.** Testpackung nach Anspruch 18, worin die Markierung ein radioaktives Element ist.

**24.** Testpackung nach Anspruch 23, worin das radioaktive Element ausgewählt ist aus der Gruppe $^{14}$C, $^{125}$I, $^{131}$I, $^{35}$S, $^{57}$Co, $^{59}$Fe und $^{3}$H.

**25.** In-vitro-Methode zur Verfolgung des Verlaufs und der Wirksamkeit einer Arzneimitteloder Diät-Therapie, worin eine Veränderung des Östrogen-Metabolismus auftritt, wobei die Methode des Anspruchs 1 durchgeführt wird, um das Verhältnis periodisch zu überwachen und irgendwelche Veränderungen festzustellen.

**Revendications**

**1.** Procédé pour détecter une pathologie chez les mammifères qui consiste:

A. à isoler au moins deux métabolites distincts de l'oestrone provenant d'un échantillon biologique prélevé chez le mammifère en examen, lesdits métabolites ayant été choisies de sorte à comparer les voies de la 2-hydroxylation et de la 16 alpha-hydroxylation du métabolisme de l'oestrone;
B. à déterminer la quantité de chacun desdits métabolites dans ledit échantillon;
C. à mettre le rapport des quantités de chacun desdits métabolites en corrélation les uns avec les autres afin de parvenir à un rapport desdits métabolites; et
D. à comparer ledit rapport avec un rapport extrinsèque déduit précédemment chez le mammifère sous examen, ou d'après les essais chez d'autres sujets de la même espèce en vue de détecter toute modification dans ledit métabolisme de l'oetrogène, lesdites modifications identifiant l'existence de ladite pathologie.

**2.** Procédé selon la revendication 1, dans lequel lesdites métabolites de l'oetrone sont choisis dans le groupe formé par les produits de l'hydroxylation enzymatique de produits d'oestrone provenant de la réduction enzymatique de l'oestrone, les produits de l'oxydation enzymatique de l'oestrone et les produits de la méthylation enzymatique de l'oestrone.

**3.** Procédé selon la revendication 1, dans lequel lesdits produits sont choisis dans le groupe formé par la 2-hydroxyoestrone, la 4-hydroxyoestrone, la 15 alphahydroxyoestrone, la 16 alpha-hydroxyoestrone, la 2-méthoxyoestrone, l'oestriol, le 17 béta-oestriol, l'épioestriol, le 16-cétooestradiol et le 15 alpha-hydroxyoestradiol.

**4.** Procédé selon la revendication 1, dans lequel lesdits produits comprennent la 2-hydroxyoestrone et l'oestriol.

**5.** Procédé selon l'une quelconque des revendications 1 a 4, dans lequel ladite concentration est mesurée au moyen d'une analyse chromatographique.

**6.** Procédé selon l'une quelconque des revendications 1 à 4, dans lequel ladite concentration est mesurée au moyen d'une analyse immunochimique.

**7.** Procédé selon l'une quelconque des revendications 1 à 4, dans lequel ladite concentration est mesurée au moyen d'une analyse radiométrique.

**8.** Procédé selon l'une quelconque des revendications 1 à 4, dans lequel ladite concentration est mesurée au moyen d'un dosage de récepteur.

9. Procédé selon la revendication 1, dans lequel ladite pathologie est choisie parmi l'ostéoporose, l'endométriose, l'obésité, l'athérosclérose, le cancer endométrial, le cancer du sein, les maladies cardiaques, l'infertilité, les maladies gastro-intestinales, les maladies du système nerveux central et leurs combinaisons.

10. Procédé pour mesurer la présence de métabolites de l'oestrone chez un mammifère, dans lequel une modification du métabolisme de l'oestrogènes est impliquée, lesdits métabolites étant choisis de sorte à comparer les voies de la 2-hydroxylation et de la 16-alpha-hydroxylation du métabolisme de l'oestrone, dans lequel chaque métabolite est mesuré:

A. en préparant au moins un anticorps qui se lie spécifiquement à un métabolite de l'oestrone;
3. en plaçant une marque détectable sur ledit métabolite ou ledit anticorps;
C. en immobilisant la matière non marquée provenant de l'étape B sur un support solide;
D. en mettant la matière marquée provenant de l'étape B et la matière immobilisée de l'étape C en contact avec un échantillon biologique provenant d'un mammifère chez lequel ladite modification du métabolisme de l'oestrogène est soupçonnée;
E. en séparant la matière marquée provenant de l'étape D qui est liée à ladite matière immobilisée d'avec la matière provenant de l'étape D non liée à ladite matière immobilisée; et
F. en examinant ladite matière liée en ce qui concerne la présence de ladite matière marquée en vue de déterminer, en se référant à un étalon, la quantité de chacun desdits métabolites de l'oestrone dans l'échantillon, en mettant les quantités de chacun desdits métabolites en corrélation les uns avec les autres afin de parvenir à un rapport desdits métabolites, et en comparant ledit rapport avec un rapport extrinsèque déduit précédemment chez le mammifère sous examen ou d'après des essais chez d'autres sujets de la même espèce afin de déterminer toute modification dudit métabolisme de l'oestrogène et de détecter l'existence d'une pathologie d'après celui-ci.

11. Procédé pour mesurer la présence des métabolites de l'oestrone chez un mammifère chez lequel une modification du métabolisme de l'oestrone est impliquée, afin de déterminer ladite modification, lesdits métabolites étant choisis de sorte à comparer les voies de la 2-hydoxylation ou de la 16-alpha-hydroxylation du métabolisme de l'oestrone, dans lequel chacun desdits métabolites est mesuré:

A. en préparant au moins un anticorps correspondant qui se lie spécifiquement à un métabolite de l'oestrone;
B. en plaçant une marque détectable sur ledit métabolite ou sur ledit anticorps de l'étape A;
C. en plaçant la matière marquée provenant de l'étape B et l'anticorps correspondant ou le métabolite qui n'est pas marqué, en contact avec un échantillon biologique chez un mammifère chez lequel ladite modification du métabolisme de l'oestrogène est soupçonnée, afin de former un mélange unitaire; et
D. en examinant la mélange de l'étape C en ce qui concerne la présence d'une activité de ladite matière marquée en vue de déterminer, en se référant à un étalon, la quantité de chacun desdits métabolites de l'oestrone dans l'échantillon, en mettant les quantités de chacun desdits métabolites en corrélation les uns avec les autres pour parvenir à un rapport desdits métabolites, et en comparant ledit rapport avec un rapport extrinsèque déduit précédemment chez le mammifère sous examen, ou d'après des essais chez d'autres sujets de la même espèce, afin de déterminer toute modification dans ledit métabolisme des oestrogènes et pour détecter l'existence d'une pathologie d'après celui-ci.

12. Procédé selon les revendications 10 ou 11, dans lequel le marqueur est une enzyme.

13. Procédé selon la revendication 12, dans lequel le marqueur est choisi dans le groupe formé par la peroxydase, la béta-glucoronidase, la bêta-D-glucosidase, la bêta-D-galactosidase, l'uréase, la glucose-oxydase plus la peroxydase, la galactose-oxydase plus la peroxydase, l'hexokinase plus la GPDase, la glucose-oxydase plus la phosphatase alcaline, la NAD-oxydoreductase plus la luciférase, la phosphofructokinase plus la pyruvate de phosphoenol-carboxylase, l'aspartate aminotransférase plus la pyruvate de phosphoenol decarboxylase, et la phosphatase alcaline.

14. Procédé selon les revendications 10 ou 11, dans lequel le marqueur est un produit chimique qui présente une fluorescence.

15. Procédé selon la revendication 14, dans lequel le produit chimique est choisi dans le groupe formé par la fluorescéine, la rhodamine et l'auramine.

16. Procédé selon les revendications 10 ou 11, dans lequel le marqueur est un élément radioactif.

17. Procédé selon la revendication 16, dans lequel l'élément radioactif est choisi dans le groupe formé par $^{14}$C, $^{125}$I, $^{131}$I, $^{35}$S, $^{57}$Co, $^{59}$Fe et $^{3}$H.

18. Trousse d'essai pour la détection d'une pathologie dans laquelle une modification du métabolisme de l'oestrogène est impliquée, utilisant le milieu sérique, tissulaire ou aqueux d'un mammifère sous examen comprenant:

> A. une quantité prédéterminée d'au moins un composant marqué immunochimiquement réactif obtenu en fixant directement ou indirectement un ou plusieurs métabolites de l'oestrone ou un partenaire de liaison spécifique à celui-ci, à un marqueur détectable, lesdits métabolites de l'oestrone étant choisis de sorte à comparer les voies de la 2-hydroxylation et de la 16-alpha-hydroxylation du métabolisme de l'oestrone;
> B. d'autres réactifs;et
> C. directives pour l'utilisation de ladite trousse.

19. Trousse d'essai selon la revendication 18, dans laquelle le marqueur est un enzyme.

20. Trousse d'essai selon la revendication 19, dans laquelle le marqueur est choisi dans le groupe formé par la peroxydase, la béta-glucuronidase, la beta-D-glucosidase, la bêta-D-galactosidase, l'uréase, la glucose-oxydase plus la peroxydase, la galactose-oxydase plus la peroxydase, l'hexokinase plus la GPDase, la glucose-oxydase plus la phosphatase alcaline, la NAD-oxydoréductase plus la luciférase, la phosphofructokinase plus la pyruvate de phosphoenol-carboxylase, l'aspartate-aminotransférase plus la pyruvate de phosphoenol-décarboxylase, et la phosphatase alcaline.

21. Trousse d'essai selon la revendication 18, dans laquelle le marqueur est un produit chimique qui présente une fluorescence.

22. Trousse d'essai selon la revendication 21, dans laquelle le produit chimique est choisi dans le groupe formé par la fluorescéine, la rhodamine et l'auramine.

23. Trousse d'essai selon la revendication 18, dans laquelle le marqueur est un élément radioactif.

24. Trousse d'essai selon la revendication 23, dans laquelle l'élément radioactif est choisi dans le groupe formé par $^{14}$C, $^{125}$I, $^{131}$I, $^{35}$S, $^{57}$Co, $^{59}$Fe et $^{3}$H.

25. Procédé in vitro pour contrôler le parcours et l'efficacité d'un médicament ou d'une thérapie diététique dans laquelle est impliquée une modification du métabolisme de l'oestrogène consistant à réaliser ie procédé selon la revendication 1 afin de contrôler périodiquement le rapport afin de noter tout changement.

FIG.I A

FIG.1B

# FIG.2 A

# FIG.2 B

% 2-HYDROXYLATION

$[2OHE_1]/[E_3]$

P < 0.001

P = 0.006

FIG.3 A

FIG.3 B

FIG.4